(19) **Europäisches Patentamt**
**European Patent Office**
**Office européen des brevets**

(11) **EP 3 454 341 A1**

(12) **EUROPÄISCHE PATENTANMELDUNG**

(43) Veröffentlichungstag:
**13.03.2019 Patentblatt 2019/11**

(51) Int Cl.:
***G16H 50/20*** *(2018.01)*

(21) Anmeldenummer: **17190561.5**

(22) Anmeldetag: **12.09.2017**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **Siemens Healthcare GmbH**
**91052 Erlangen (DE)**

(72) Erfinder:
- **Ionasec, Razvan**
  **90402 Nuernberg (DE)**
- **Schmidt, Sebastian**
  **91085 Weisendorf (DE)**
- **Shah, Amitkumar Bhupendrakumar**
  **91052 Erlangen (DE)**

(54) **AUTOMATISIERTES VERARBEITEN VON PATIENTENDATEN ZUR GESUNDHEITSBETREUUNG**

(57)      Es wird ein Verfahren zur Qualifizierung von Patienten für eine medizinische Behandlung (BV) oder Untersuchung (UV) beschrieben. Bei dem Verfahren werden biologische Daten (MD) von Mitgliedern eines Kollektivs potentieller Patienten sensoriell erfasst. Weiterhin werden die erfassten biologischen Daten (MD) per Funk an eine zentrale Auswerteeinheit (5) übertragen. Es erfolgt zudem ein automatisiertes Ermitteln eines oder mehrerer Risikowerte (RW) für eine oder mehrere Arten von Krankheiten für die Mitglieder (2) des Kollektivs auf Basis der biologischen Daten (MD). Schließlich werden einzelne Mitglieder (2) Untersuchungen (UV) oder Behandlungen (BV) in Abhängigkeit von dem jeweils ermittelten Risikowert (RW) zugeordnet. Es wird auch ein Qualifizierungssystem (1) beschrieben.

FIG 1

EP 3 454 341 A1

**Beschreibung**

Die Erfindung betrifft ein Verfahren zur Qualifizierung von Patienten für eine medizinische Behandlung oder Untersuchung. Weiterhin betrifft die Erfindung ein Qualifizierungssystem.

Für eine effektive Gesundheitsbetreuung der Bevölkerung und eine präventive Gesundheitsstrategie ist es wichtig, relevante Daten auf kostengünstige Weise von der gesamten betroffenen Population zu beschaffen und Vorhersageregeln zu etablieren, mit denen Risikogruppen identifiziert werden können, um diese einer eingehenderen Untersuchung, zum Beispiel einer diagnostischen Bildgebung zuzuführen.

Der Erfolg jedes Massen-Screening-Programms hängt sehr stark von der den Patienten zugeordneten Wahrscheinlichkeit für einen positiven Befund ab. In diesem Zusammenhang gibt der positive Vorhersagewert PPV an, wie viele Personen der Untersuchten, bei denen ein positiver Befund ermittelt wurde, auch tatsächlich von einer gesuchten Krankheit betroffen sind. Der positive Vorhersagewert hängt von der Prävalenz prev, der Spezifizität spec und der Sensitivität sens ab und ergibt sich zu:

$$PPV = \frac{sens \cdot prev}{sens \cdot prev + (1 - spec) \cdot (1 - prev)} . \qquad (1)$$

Dabei gibt die Prävalenz prev die Häufigkeit der tatsächlich kranken Personen in einem untersuchten Kollektiv an. Die Spezifizität spec gibt die Wahrscheinlichkeit an, dass tatsächlich Gesunde im Test auch als gesund erkannt werden. Die Sensitivität dagegen gibt die Wahrscheinlichkeit eines Tests an, dass tatsächlich kranke Personen auch als krank identifiziert werden.

Wenn also die Prävalenz prev sehr niedrig ist, ist der positive Vorhersagewert PPV sehr niedrig, auch wenn der Test eine sehr gute technische Leistungsfähigkeit, d.h. eine hohe Sensitivität sens und Spezifizität spec, aufweist. Bei einem niedrigen positiven Vorhersagewert PPV ergibt sich eine hohe Anzahl von falsch positiven Ergebnissen und daraus resultierenden unnötigen Interventionen. Daher ist die Auswahl der richtigen Zielgruppe für jedes Screening-Programm entscheidend.

Aus diesem Grund sind Screening-Programme auf spezielle Gruppen, definiert durch Alter, Geschlecht, Lebensgewohnheiten usw. beschränkt. Diese Einschlusskriterien sind sehr grob und leider nicht ausreichend. Beispielsweise treten nur 30 % der Lungenkrebsfälle in der Gruppe auf, welche aufgrund der genannten groben Kriterien für das Screening ausgewählt wurde. Andere mögliche Screening-Programme, wie zum Beispiel NT-proBNP Screening (NT-proBNP = N terminal pro B Natriurectic Peptide = natriuretisches Peptid Typ B) für Herzinsuffizienz, werden gar nicht erst umgesetzt, weil bei ihnen keine geeignete Definition einer Zielgruppe existiert.

Um Personen für ein solches Screening-Programm zu qualifizieren, das zum Beispiel auf einer medizinischen Bildgebung basiert, wie zum Beispiel ein Screening für Lungenkrebs, werden Daten, wie zum Beispiel das Alter, die Rauchgewohnheiten, die Familiengeschichte und konkrete Symptome, verwendet, um Risikoberechnungen vorzunehmen, die für eine Entscheidung bezüglich einer weiteren klinischen Betreuung, die zum Beispiel eine Bildgebung oder andere Tests vorsieht, genutzt werden.

Es gibt zwei grundlegende Probleme bei Volksgesundheitsmanagementsystemen (im Englischen "population management system") bezüglich des Auffindens und Qualifizierens von Personen für präventive Screening-Programme, welche im Folgenden beschrieben werden.

Ein derartiges Volksgesundheitsverwaltungssystems (im englischen "population health management system") kann beispielsweise die Verwaltung bzw. optimierte Nutzung von Daten für eine bestimmte "Population" von Individuen, z.B. ein Volk, eine bestimmte Bevölkerungsgruppe oder ein bestimmtes Patientenkollektiv betreffen. Der Begriff "Volk" kann sich dabei auf die Staatsangehörigen eines bestimmten Staates oder einer Gruppe von Staaten (z.B. Mitgliedsstaaten der Europäischen Union), auf die Mitglieder einer bestimmten ethnischen Gruppe oder auf die Mitglieder eines bestimmten Gesundheitssystems beziehen. Der Begriff "Bevölkerungsgruppe" kann sich dabei beispielsweise auf eine Untergruppe eines "Volkes" beziehen, z.B. Personen oberhalb oder unterhalb eines bestimmten Alters, Personen eines bestimmten Geschlechts, o.ä.). Der Begriff "Patientenkollektiv" kann sich dabei auf eine Gruppe von Individuen mit einer bestimmten Krankheitsdiagnose beziehen, z.B. eine Gruppe von Diabetes-Patienten, eine Gruppe von Bluthochdruck-Patienten, o.ä.).

Zum einen muss die richtige Menge von Daten und der richtige Datentyp gefunden werden. Diese Daten sollten als Basis für die Risikoberechnung mit einer ausreichenden Vorhersagekraft für die einzelnen Personen dienen. Aktuell werden nur Basisdaten, wie zum Beispiel das Alter, das Geschlecht, Kernrisikofaktoren und historische Daten für die Risikoberechnung verwendet. Infolge der ungenauen Eingangsdaten sind solche Modelle oft nicht in der Lage, Personen mit höheren Risiken vorherzusagen.

Zweitens werden Daten für die gesamte Population benötigt. Die erfassten Daten sollten vollständig für die gesamte Bevölkerung vorliegen, um eine Risikoberechnung für alle Personen vornehmen zu können. Aktuell stehen nur Daten für eine Untergruppe von Personen zur Verfügung, die aus verschiedenen Quellen stammen und eine stark variierende Qualität und Korrektheit aufweisen.

Es besteht also dass Problem, Daten für eine verlässlichere Vorhersage für ein Krankheitsrisiko von Patienten zu bekommen, um geeignete Personen für eine Vorsorgeuntersuchung, wie zum Beispiel ein Screening-Programm, zu qualifizieren, d.h. bei einer solchen Vorsorgeuntersuchung einen verbesserten positiven Vorhersagewert zu erhalten.

Ein weiteres Problem kann darin bestehen, dass ein teures oder risikoreiches Heilverfahren nur auf Patienten angewandt werden soll, bei denen es ein gutes Chance/Risiko-Verhältnis bzw. ein gutes Chance/Kosten-Verhältnis aufweist. Um den geeigneten Patientenkreis auszuwählen, müssen vorab entsprechende statistische Informationen aus einem größeren Personenkreis ermittelt werden.

Die genannten Probleme werden durch ein Verfahren zur Qualifizierung von Patienten für eine medizinische Behandlung oder Untersuchung gemäß Patentanspruch 1 und durch ein Qualifizierungssystem gemäß Patentanspruch 7 gelöst.

Bei dem erfindungsgemäßen Verfahren zur Qualifizierung von Patienten für eine medizinische Behandlung oder Untersuchung werden biologische Daten von Mitgliedern eines Kollektivs potentieller Patienten sensoriell erfasst. Biologische Daten sollen Daten umfassen, welche den menschlichen Körper und dessen Funktion betreffen, beispielsweise die Körpertemperatur, aber auch die Anzahl der von einem Menschen zurückgelegten Schritte, umfassen. Insbesondere sollen biologische Daten auch physiologische Daten, also Daten, welche physikalische und/oder biochemische Prozesse in Zellen, Geweben und Organen von Personen betreffen, umfassen. Weiterhin sollen biologische Daten medizinische Daten, also den Gesundheitszustand von Personen betreffende Daten bzw. gesundheitlich relevante Daten umfassen. Medizinische Daten können zum Beispiel die Herzfrequenz, die Körpertemperatur, das Schlafverhalten, das Bewegungsmuster, den Blutdruck, EKG-Daten, die Sauerstoffkonzentration des Blutes, den Blutzuckerspiegel und Daten bezüglich des Alkoholkonsums und des Nikotinkonsums umfassen. Es können auch zusätzliche Parameter von Kamerabildern, vitale Körperfunktionen betreffende Parameter, der BMI, das Alter, das Geschlecht, das Gewicht, die Art und Menge der konsumierten Medikamente, andere entdeckte Krankheiten, die medizinische Historie bzw. Krankengeschichte von Patienten mit berücksichtigt werden.

Zum Erfassen der gewünschten Daten können zum Beispiel am Körper oder an der Kleidung der Mitglieder des Kollektivs entsprechende Sensoren befestigt sein, die die biologischen Daten erfassen. Die derart ermittelten biologischen Daten werden dann per Funk an eine zentrale Auswerteeinheit übermittelt.

Dort werden ein oder mehrere Risikowerte für eine oder mehrere Arten von Krankheiten für die Mitglieder des Kollektivs auf Basis der biologischen Daten automatisiert ermittelt. Schließlich werden einzelne Mitglieder des Kollektivs in Abhängigkeit von dem jeweils ermittelten Risikowert Untersuchungen oder Behandlungen zugeordnet. Vorteilhafter Weise werden hierdurch potentielle Patienten die von einer Vorsorgemaßnahme oder einer Behandlung besonders profitieren würden, identifiziert. Dadurch wird die Prävalenz bestimmter zu detektierender Krankheiten oder Dispositionen erhöht und damit der positive Vorhersagewert drastisch verbessert. Konkret wird in Folge der erhöhten Prävalenz insbesondere das Verhältnis der Anzahl der korrekten positiven Befunde zu der Anzahl der falsch-positiven Befunde deutlich verbessert, so dass für Vorsorgemaßnahmen oder gar Behandlungen eine erhöhte Akzeptanz und ein verbesserter Nutzen erreicht werden kann.

Das erfindungsgemäße Qualifizierungssystem weist eine Mehrzahl von Sensoren auf, welche an Mitgliedern eines Kollektivs potentieller Patienten angeordnet sind und dazu dienen, biologische Daten von den potentiellen Patienten sensoriell zu erfassen. Teil des erfindungsgemäßen Qualifizierungssystems sind auch eine Mehrzahl von Funkeinheiten zum Übertragen der erfassten biologischen Daten per Funk an eine zentrale Auswerteeinheit. Als Funkeinheiten können zum Beispiel mobile Endgeräte, wie zum Beispiel Mobiltelefone der Mitglieder des Kollektivs, genutzt werden. Das erfindungsgemäße Qualifizierungssystem umfasst zudem eine zentrale Auswertungseinheit zum automatisierten Ermitteln eines oder mehrerer Risikowerte für eine oder mehrere Arten von Krankheiten für die Mitglieder des Kollektivs auf Basis der biologischen Daten. Die zentrale Auswertungseinheit empfängt also die dezentral erfassten Sensordaten der Mitglieder des Kollektivs und wertet diese im Hinblick auf Risiken für eine oder mehrere Arten von Krankheiten aus. Das erfindungsgemäße Qualifizierungssystem umfasst auch eine Zuordnungseinheit zum Zuordnen einzelner Mitglieder zu Untersuchungen oder Behandlungen in Abhängigkeit von dem jeweils ermittelten Risikowert. Das erfindungsgemäße Qualifizierungssystem teilt die Vorteile des erfindungsgemäßen Verfahrens zur Qualifizierung von Patienten für eine medizinische Behandlung oder Untersuchung.

Einige Komponenten des erfindungsgemäßen Qualifizierungssystems können zum überwiegenden Teil in Form von Softwarekomponenten ausgebildet sein. Dies betrifft insbesondere Teile der zentralen Auswertungseinheit und der Zuordnungseinheit. Grundsätzlich können diese Komponenten aber auch zum Teil, insbesondere wenn es um besonders schnelle Berechnungen geht, in Form von softwareunterstützter Hardware, beispielsweise FPGAs oder dergleichen, realisiert sein. Ebenso können die benötigten Schnittstellen, beispielsweise wenn es nur um eine Übernahme von Daten aus anderen Softwarekomponenten geht, als Softwareschnittstellen ausgebildet sein. Sie können aber auch als hardwaremäßig aufgebaute Schnittstellen ausgebildet sein, die durch geeignete Software angesteuert werden.

Eine weitgehend softwaremäßige Realisierung hat den Vorteil, dass auch schon bisher für medizinische Aufgaben genutzte Rechnersysteme auf einfache Weise durch ein Software-Update nachgerüstet werden können, um auf die erfindungsgemäße Weise als Qualifizierungssystem zu arbeiten. Um die vollständigen Funktionen des erfindungsgemäßen Qualifizierungssystems bieten zu können, muss allerdings eine zusätzliche Nachrüstung von Hardwareelementen, wie zum Beispiel die an den potentiellen Patienten angeordneten Sensoren und Funkeinheiten erfolgen. Insofern wird die Aufgabe auch durch ein entsprechendes Computerprogrammprodukt mit einem Computerprogramm gelöst, welches direkt in eine Speichereinrichtung eines Rechnersystems ladbar ist, mit Programmabschnitten, um alle Schritte des erfindungsgemäßen Verfahrens auszuführen bzw. zumindest zu steuern, wenn das Computerprogramm in der Rechnereinheit ausgeführt wird. Während die Auswerteschritte und Zuordnungsschritte direkt in der Rechnereinheit erfolgen können, können zum Beispiel das Sammeln der Sensordaten sowie das Übertragen per Funk an die Rechnereinheit von der Rechnereinheit aus durch eine entsprechende Kommunikation mit patientenseitig gespeicherten Programmteilen überwacht und gesteuert werden. Das Computerprogramm kann also auch über mehrere Einheiten verteilt gespeichert sein und das Rechnersystem ein Netzwerk von Rechnern bzw. prozessierenden Einheiten umfassen, welche zur Realisierung des erfindungsgemäßen Verfahrens zusammenarbeiten.

Ein solches Computerprogrammprodukt kann neben dem Computerprogramm gegebenenfalls zusätzliche Bestandteile wie z.B. eine Dokumentation und/oder zusätzliche Komponenten auch Hardware-Komponenten, wie z.B. Hardware-Schlüssel (Dongles etc.) zur Nutzung der Software, umfassen.

Zum Transport zu einer Speichereinrichtung des Rechnersystems und/oder zur Speicherung an einer oder mehreren solchen Einrichtungen kann ein computerlesbares Medium, beispielsweise ein Memorystick, eine Festplatte oder ein sonstiger transportabler oder fest eingebauter Datenträger dienen, auf welchem die von dem Rechnersystem einlesbaren und ausführbaren Programmabschnitte des Computerprogramms gespeichert sind. Das Rechnersystem kann zum Beispiel hierzu einen oder mehrere zusammenarbeitende Mikroprozessoren oder dergleichen aufweisen.

Die abhängigen Ansprüche sowie die nachfolgende Beschreibung enthalten jeweils besonders vorteilhafte Ausgestaltungen und Weiterbildungen der Erfindung. Dabei können insbesondere die Ansprüche einer Anspruchskategorie auch analog zu den abhängigen Ansprüchen einer anderen Anspruchskategorie und deren Beschreibungsteilen weitergebildet sein. Zudem können im Rahmen der Erfindung auch die verschiedenen Merkmale unterschiedlicher Ausführungsbeispiele und Ansprüche auch zu neuen Ausführungsbeispielen kombiniert werden.

In einer Ausgestaltung des erfindungsgemäßen Verfahrens umfassen die medizinischen Untersuchungen, für die potentielle Patienten qualifiziert werden, ein medizinisches Bildgebungsverfahren. Medizinische Bildgebungsverfahren können zum Beispiel im Rahmen von sogenannten Screening-Verfahren eingesetzt werden. Vorteilhaft kann mit Hilfe des erfindungsgemäßen Verfahrens ein geeignetes Kandidatenkollektiv für ein Screening-Verfahren gefunden werden, welches eine hohe Prävalenz für eine mit Hilfe des Screening-Verfahrens aufzufindende Krankheit haben. Vorteilhaft wird auf diese Weise der positive Vorhersagewert und damit die Akzeptanz eines solchen Screening-Verfahrens verbessert.

In vielen Gebieten werden Verfahren mit Lernalgorithmen, wie zum Beispiel "deep learning" oder "machine learning" angewandt. Eine große Anzahl von Datensätzen mit bekannten Informationen werden verwendet, um eine Software zu trainieren, die dann dazu in der Lage ist, vorher unbekannte Datensätze zu analysieren. Diese Verfahren werden aktuell breit angewandt für Aufgaben in der Bildanalyse, wie zum Beispiel Personenerkennung oder bei selbstfahrenden Fahrzeugen.

In einer Ausgestaltung des erfindungsgemäßen Verfahrens zur Qualifizierung von Patienten für eine medizinische Behandlung oder Untersuchung wird nun für das automatisierte Ermitteln eines oder mehrerer Risikowerte ein durch ein solches maschinelles Lernverfahren trainiertes Ermittlungsverfahren eingesetzt. Ein solches maschinelles Lernverfahren umfasst einen vorab durchgeführten Trainingsprozess, in dem mit Hilfe eines Trainings-Datensatzes ein gewünschtes Auswerteverhalten trainiert wird.

Die Auswerteergebnisse des Trainingsdatensatzes sind vorab bekannt und können für eine schrittweise Anpassung des Auswerteprozesses genutzt werden. Die Sensordaten stellen eine Zeitserie von Daten dar, welche mit Hilfe von LSTM-Techniken (LSTM = long short term memory) in rekurrenten neuronalen Netzwerk-Deep-Learning-Architekturen angewandt werden können. Mit diesen speziellen Techniken lassen sich Gradienten bei der Ausführung eines Rückpropagationsalgorithmus auf Werte (insbesondere Werte $> 0$ und $< \infty$) begrenzen, mit denen ein Anpassungsvorgang des Auswerteprozesses vorgenommen werden kann.

Zum Beispiel können bei dem Training der Detektion von Risiken für Herzkrankheiten EKGs zusammen mit klinischen Befunden anhand von Bilddaten und anderen Tests ebenso wie andere relevante EHR-Daten (EHR = electronic health record = über Netzwerke austauschbare medizinische Daten von Patienten) derselben Person berücksichtigt werden. Zusätzlich können auch Informationen aus der medizinischen Literatur und Expertenmeinungen als Information für die Trainingsphase genutzt werden.

In der Detektionsphase wird dann das gelernte statistische Modell auf die erfassten Sensordaten angewandt, um einen Risikowert eines potentiellen Patienten für eine Krankheit bzw. einen positiven Befund bei einer Vorsorgeuntersuchung abzuschätzen. Vorteilhaft lässt sich ein Auswertungsvorgang mit Hilfe eines maschinellen Lernverfahrens an unterschiedliche medizinische Fragestellungen anpassen und erhöht damit die Flexibilität eines Auswertevorgangs bzw. der dafür verwendeten Einrichtungen und Computerprogramme.

In einer Ausgestaltung des erfindungsgemäßen Verfahrens zur Qualifizierung von Patienten für eine medizinische Behandlung oder Untersuchung wird bei dem maschinellen Lernverfahren ein rekurrentes neuronales Netz eingesetzt. Als rekurrente bzw. rückgekoppelte neuronale Netze bezeichnet man neuronale Netze, die sich, im Gegensatz zu den sogenannten Feedforward-Netzen, durch Verbindungen von Neuronen einer Schicht zu Neuronen derselben oder einer vorangegangenen Schicht auszeichnen. In künstlichen neuronalen Netzen werden rekurrente Verschaltungen von Modellneuronen benutzt, um zeitlich kodierte Informationen in den Daten zu entdecken. Praktische Anwendung finden rekurrente neuronale Netze bei Problemstellungen, die das Verarbeiten von Sequenzen erfordern. Die hierbei vorherrschende Art der rekurrenten neuronalen Netze sind LSTMs, beziehungsweise ähnliche Varianten, die auf einer direkten Rückkopplung basieren.

In einer besonders bevorzugten Variante des erfindungsgemäßen Verfahrens zur Qualifizierung von Patienten für eine medizinische Behandlung oder Untersuchung werden auf Basis der erfassten biologischen Daten medizinische Behandlungen der einzelnen Mitglieder spezifiziert. Vorteilhaft können die erfassten biologischen Daten zur Anpassung eines Behandlungsprozesses an individuelle biologische bzw. medizinische Parameter eines Patienten genutzt werden, die sonst über eine entsprechende Befragung oder einen Zugriff auf Datenbanken gesammelt werden müssten.

Die bei dem erfindungsgemäßen Verfahren zur Qualifizierung von Patienten für eine medizinische Behandlung oder Untersuchung verwendeten Sensoren können zum Beispiel von tragbaren Geräten, sogenannte "Wearable Devices", auch "Wearables" genannt, umfasst sein. Diese Geräte umfassen sogenannte "Smart Watches", "Smart Wristbands", "Smart Patches, Kontaktlinsen, Brillen usw., die ununterbrochen oder von Zeit zu Zeit gesundheitlich relevante Daten erfassen.

Die Erfindung wird im Folgenden unter Hinweis auf die beigefügten Figuren anhand von Ausführungsbeispielen noch einmal näher erläutert. Es zeigen:

FIG 1 ein Flussdiagramm, welches ein Verfahren zum Qualifizieren von Patienten für eine medizinische Behandlung oder Untersuchung gemäß einem Ausführungsbeispiel der Erfindung veranschaulicht,

FIG 2 ein Blockdiagramm, mit dem ein Qualifizierungssystem gemäß einem Ausführungsbeispiel der Erfindung dargestellt wird.

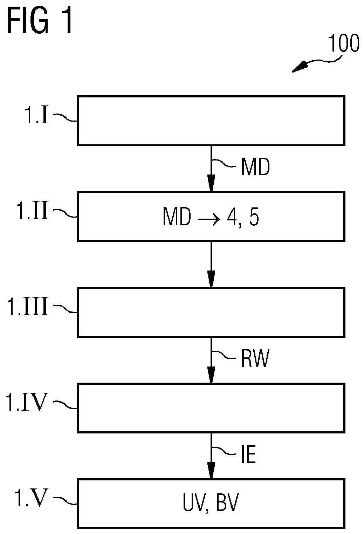

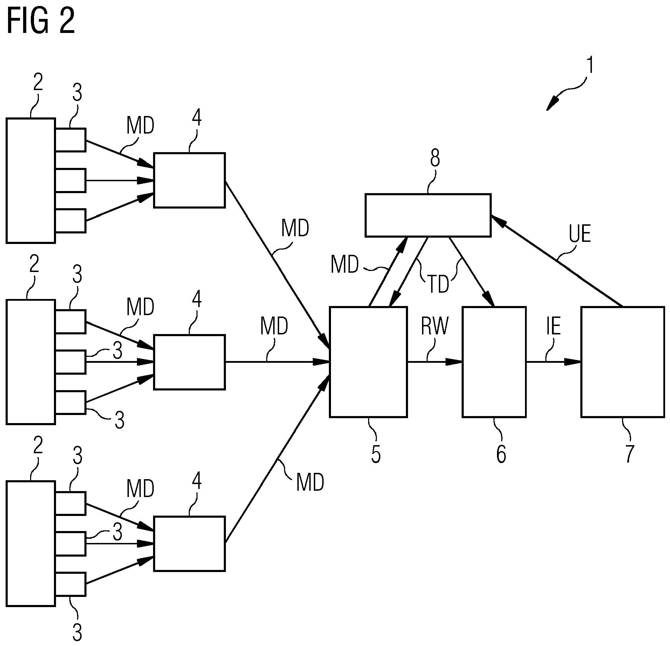

In FIG 1 ist ein Flussdiagramm 100 gezeigt, welches ein Verfahren zum Qualifizieren von Patienten für eine medizinische Behandlung oder Untersuchung veranschaulicht. Bei dem Schritt 1.I werden zunächst über einen bestimmten Zeitraum Sensordaten MD mit Hilfe von Sensoren 3 (siehe FIG 2) von potentiellen Patienten 2 (siehe FIG 2) bzw. Mitgliedern eines Kollektivs erfasst. Bei dem Schritt 1.II werden die Sensordaten MD von einer an den Mitgliedern des Kollektivs angebrachten und mit den Sensoren elektrisch verbundenen Funkeinheiten 4 (siehe FIG 2) an eine zentrale Auswertungseinheit 5 (siehe FIG 2) übertragen. Von der zentralen Auswertungseinheit 5 werden bei dem Schritt 1.III für jedes der Mitglieder 2 des Kollektivs automatisiert Risikowerte RW für eine Anzahl von Krankheiten auf Basis der Sensordaten MD ermittelt. Bei dem Schritt 1.IV werden nachfolgend auf Basis der Risikowerte RW automatisiert Entscheidungen IE über die Zuordnung der Mitglieder 2 zu Vorsorgeprogrammen oder Behandlungsprozessen gefällt. Anschließend werden bei dem Schritt 1.V Untersuchungsvorgänge UV, wie zum Beispiel Screening-Prozesse, und/oder Behandlungsprozesse BV bei den entsprechend zugeordneten Mitgliedern 2 des Kollektivs auf Basis der Entscheidungsinformationen IE gestartet.

In FIG 2 ist ein Qualifizierungssystem 1 gemäß einem Ausführungsbeispiel der Erfindung veranschaulicht. Das Qualifizierungssystem umfasst eine Mehrzahl von Sensoren 3, welche an Mitgliedern 2 eines Kollektivs potentieller Patienten angeordnet sind. Die Sensoren 3 können zum Beispiel direkt an der Kleidung der überwachten Personen 2 oder auf deren Körper befestigt sein. Die Sensoren 3 dienen dazu, biologische, insbesondere medizinische Daten MD von den potentiellen Patienten 2 sensoriell zu erfassen. Das Qualifizierungssystem 1 umfasst auch eine Mehrzahl von jeweils den einzelnen Mitgliedern 2 des Kollektivs zugeordneten Funkeinheiten 4, mit denen die sensoriell erfassten medizinischen Daten MD per Funk an eine zentrale Auswerteeinheit 5 übertragen werden, die ebenfalls Teil des Qualifizierungssystems 1 ist.

Die zentrale Auswertungseinheit 5 dient dazu, Risikowerte RW für ein positives Ergebnis bei einem Screening-Verfahren oder Wahrscheinlichkeitswerte für eine Eignung eines Patienten für eine bestimmte Therapie zu ermitteln. Die Ergebnisse RW der Auswertung werden an eine Zuordnungseinheit 6 übermittelt, welche auf Basis der ermittelten Ergebnisse RW eine Entscheidung darüber trifft, ob ein Mitglied 2 des Kollektivs für ein Screeningverfahren oder für ein bestimmtes Behandlungsverfahren als geeignet eingestuft wird. Das Entscheidungsergebnis IE wird an eine Behandlungseinrichtung 7 übermittelt, welche unterschiedliche Einheiten und Vorrichtungen zur Vorsorge und/oder Therapie der Mitglieder des Kollektivs bereithält. Die bei den Untersuchungen in der Behandlungseinrichtung 7 ermittelten Untersuchungsergebnisse UE werden in einer Trainingsdatenbank 8 abgespeichert.

Den Untersuchungsergebnissen UE zugeordnet werden auch die für den jeweiligen Patienten sensoriell erfassten medizinischen Daten MD in der Trainingsdatenbank 8 abgespeichert. Mit Hilfe der in der Trainingsdatenbank 8 gespeicherten Trainingsdaten TD können in einem Trainingsverfahren die Auswertungseinheit 5 und die Zuordnungseinheit 6 trainiert werden. Dabei werden zum Beispiel die in der Trainingsdatenbank 8 gespeicherten medizinischen Daten MD zu einer Vielzahl von Patients 2, welche vergleichbare Sensordaten MD aufweisen, an die Auswertungseinheit 5 übermittelt. Die Auswertungseinheit 5 erzeugt trainingshalber dazu Risikowerte RW, die an die Zuordnungseinheit 6 übermittelt werden. Die Risikowerte RW können dann mit der tatsächlichen Verteilung der in der Behandlungseinrichtung 8 detektierten Krankheiten bzw. dem tatsächlichen Risiko der Patienten, dass bei Ihnen eine der untersuchten Krankheiten entdeckt wurde, verglichen werden. Bei einer vorhandenen Abweichung der Risikowerte RW von den durch die Trainingsdatenbank definierten Referenzwerten, kann die Berechnung der Risikowerte mit Hilfe eines neuronalen Netzes entsprechend angepasst werden, bis die bei dem Trainingsvorgang errechneten Risikowerte RW der tatsächlichen Risikoverteilung der Trainingsdaten TD entsprechen.

Es wird abschließend noch einmal darauf hingewiesen, dass es sich bei den vorbeschriebenen Verfahren und Vorrichtungen lediglich um bevorzugte Ausführungsbeispiele der Erfindung handelt und dass die Erfindung vom Fachmann variiert werden kann, ohne den Bereich der Erfindung zu verlassen, soweit er durch die Ansprüche vorgegeben ist. So wurden das Verfahren und die Überwachungseinrichtung in erster Linie in Bezug auf eine Eignung von Patienten für ein Screening-Verfahren beschrieben. Die Erfindung ist jedoch nicht auf diese konkrete Anwendung beschränkt, sondern die Erfindung kann auch grundsätzlich auf eine Vielzahl unterschiedlicher medizinischer Fragestellungen, welche die Eignung von medizinischen Maßnahmen für einzelne Patienten oder bestimmte Patientengruppe betreffen, angewandt werden. Es wird der Vollständigkeit halber auch darauf hingewiesen, dass die Verwendung der unbestimmten Artikel "ein" bzw. "eine" nicht ausschließt, dass die betreffenden Merkmale auch mehrfach vorhanden sein können. Ebenso schließt der Begriff "Einheit" nicht aus, dass diese aus mehreren Komponenten besteht, die gegebenenfalls auch räumlich verteilt sein können.

## Patentansprüche

1. Verfahren zur Qualifizierung von Patienten für eine medizinische Behandlung (BV) oder Untersuchung (UV), aufweisend die Schritte:

    - sensorielles Erfassen von biologischen Daten (MD) von Mitgliedern (2) eines Kollektivs potentieller Patienten,
    - Übertragen der erfassten biologischen Daten (MD) per Funk an eine zentrale Auswerteeinheit (5),
    - automatisiertes Ermitteln eines oder mehrerer Risikowerte (RW) für eine oder mehrere Arten von Krankheiten für die Mitglieder (2) des Kollektivs auf Basis der biologischen Daten (MD),
    - Zuordnen einzelner Mitglieder (2) zu medizinischen Untersuchungen (UV) oder Behandlungen (BV) in Abhängigkeit von dem jeweils ermittelten Risikowert (RW).

2. Verfahren nach Anspruch 1, wobei die medizinischen Untersuchungen (UV) ein medizinisches Bildgebungsverfahren umfassen.

3. Verfahren nach Anspruch 2, wobei das medizinische Bildgebungsverfahren ein Screening-Verfahren umfasst.

4. Verfahren nach einem der vorstehenden Ansprüche, wobei für das automatisierte Ermitteln eines oder mehrerer Risikowerte (RW) ein durch ein maschinelles Lernverfahren trainiertes Ermittlungsverfahren eingesetzt wird.

5. Verfahren nach Anspruch 4, wobei bei dem maschinellen Lernverfahren ein rekurrentes neuronales Netz eingesetzt wird.

6. Verfahren nach einem der vorstehenden Ansprüche, wobei auf Basis der erfassten biologischen Daten (MD) medizinische Behandlungen der einzelnen Mitglieder (2) spezifiziert werden.

7. Qualifizierungssystem (1), aufweisend:

    - eine Mehrzahl von Sensoren (3), welche an Mitgliedern (2) eines Kollektivs potentieller Patienten angeordnet sind, zum sensoriellen Erfassen von medizinischen Daten (MD),
    - eine Mehrzahl von Funkeinheiten (4) zum Übertragen der erfassten biologischen Daten (MD) per Funk an eine zentrale Auswertungseinheit (5),
    - eine zentrale Auswertungseinheit (5) zum automatisierten Ermitteln eines oder mehrerer Risikowerte (RW) für eine oder mehrere Arten von Krankheiten für die Mitglieder (2) des Kollektivs auf Basis der biologischen Daten (MD),
- eine Zuordnungseinheit (6) zum Zuordnen einzelner Mitglieder (2) zu Untersuchungen (UV) oder Behandlungen (BV) in Abhängigkeit von dem jeweils ermittelten Risikowert (RW).

**8.** Qualifizierungssystem (1) nach Anspruch 7, wobei die Funkeinheiten (4) in Mobiltelefonen der Mitglieder (2) des Kollektivs angeordnet sind.

**9.** Computerprogrammprodukt mit einem Computerprogramm, welches direkt in eine Speichereinheit eines Rechnersystems ladbar ist, mit Programmabschnitten, um alle Schritte des Verfahrens nach einem der Ansprüche 1 bis 6 auszuführen, wenn das Computerprogramm in dem Rechnersystem ausgeführt wird.

**10.** Computerlesbares Medium, auf welchem von einer Rechnereinheit eines Qualifizierungssystems (1) ausführbare Programmabschnitte gespeichert sind, um alle Schritte des Verfahrens nach einem der Ansprüche 1 bis 6 auszuführen, wenn die Programmabschnitte von der Rechnereinheit ausgeführt werden.

FIG 1

100

1.I

MD

1.II  MD → 4, 5

1.III

RW

1.IV

IE

1.V  UV, BV

# FIG 2

Europäisches Patentamt
European Patent Office
Office européen des brevets

# EUROPÄISCHER RECHERCHENBERICHT

Nummer der Anmeldung

EP 17 19 0561

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | Jennifer Bresnick: "Using Risk Scores, Stratification for Population Health Management", 7. August 2017 (2017-08-07), XP055452594, Gefunden im Internet: URL:web.archive.org/web/20170807141739/https://healthitanalytics.com/features/using-risk-scores-stratification-for-population-health-management [gefunden am 2018-02-22] | 1-3,6-10 | INV. G16H50/20 |
| Y | * Artikelüberschrift; Seite 1/9 * * Seite 2/9 * * Seite 5/9, Absatz 3 * * Seite 6/9, Absätze 4, 11 * & Jennifer Bresnick: "Explaining the Basics of the Internet of Things for Healthcare", 30. Juni 2017 (2017-06-30), XP055452999, Gefunden im Internet: URL:https://web.archive.org/web/20170630033244/https://healthitanalytics.com/features/explaining-the-basics-of-the-internet-of-things-for-healthcare [gefunden am 2018-02-22] * Seite 2/6, Absätze 5, 6 * * Seite 3/6, Absatz 1 - Absatz 4 * -/-- | 4,5 | |

**RECHERCHIERTE SACHGEBIETE (IPC)**

G16H

Der vorliegende Recherchenbericht wurde für alle Patentansprüche erstellt

| Recherchenort | Abschlußdatum der Recherche | Prüfer |
|---|---|---|
| München | 5. März 2018 | Heidrich, Alexander |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
...........................................................
& : Mitglied der gleichen Patentfamilie, übereinstimmendes Dokument

EPO FORM 1503 03.82 (P04C03)

Seite 1 von 2

Europäisches
Patentamt

European
Patent Office

Office européen
des brevets

# EUROPÄISCHER RECHERCHENBERICHT

Nummer der Anmeldung

EP 17 19 0561

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| | & Jennifer Bresnick: "Top 4 Machine Learning Use Cases for Healthcare Providers", , 5. September 2017 (2017-09-05), XP055453390, Gefunden im Internet: URL:https://web.archive.org/web/20170905023314/https:/healthitanalytics.com/news/top-4-machine-learning-use-cases-for-healthcare-providers [gefunden am 2018-02-22] * Abschnitt "Clinical decision support and predictive analytics"; Seite 3/5 - Seite 4/5 * ----- | | |
| Y | TOM QUISEL ET AL: "Collecting and Analyzing Millions of mHealth Data Streams", KNOWLEDGE DISCOVERY AND DATA MINING, ACM, 2 PENN PLAZA, SUITE 701NEW YORKNY10121-0701USA, 13. August 2017 (2017-08-13), Seiten 1971-1980, XP058370768, DOI: 10.1145/3097983.3098201 ISBN: 978-1-4503-4887-4 * das ganze Dokument * ----- | 4,5 | RECHERCHIERTE SACHGEBIETE (IPC) |
| Y | Zachary C Lipton ET AL: "Learning to Diagnose with LSTM Recurrent Neural Networks", arXiv:1511.03677v7 [cs.LG], 21. März 2017 (2017-03-21), XP055453462, Gefunden im Internet: URL:https://arxiv.org/pdf/1511.03677v7.pdf [gefunden am 2018-02-22] * das ganze Dokument * ----- | 4,5 | |

Der vorliegende Recherchenbericht wurde für alle Patentansprüche erstellt

| Recherchenort | Abschlußdatum der Recherche | Prüfer |
|---|---|---|
| München | 5. März 2018 | Heidrich, Alexander |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes
Dokument

EPO FORM 1503 03.82 (P04C03)